# EUROPÄISCHE PATENTSCHRIFT

(11) **EP 1 047 396 B1**
(45) Veröffentlichungstag und Bekanntmachung des Hinweises auf die Patenterteilung: **14.08.2002**
(21) Anmeldenummer: 99901592.8
(22) Anmeldetag: 09.01.1999
(51) Int. Cl.: A61K 7/48

(54) **LIPOPROTEIN-CREMES**
LIPOPROTEIN CREAMS
CREMES AUX LIPOPROTEINES

(30) Priorität: 17.01.1998 DE 19801593
(43) Veröffentlichungstag der Anmeldung: 02.11.2000
(73) Patentinhaber: Henkel Kommanditgesellschaft auf Aktien, 40589 Düsseldorf-Holthausen (DE)
(72) Erfinder: WALDMANN-LAUE, Marianne, D-40789 Monheim (DE); FÖRSTER, Thomas, D-40699 Erkrath (DE); HEINEN, Soraya, D-50935 Köln (DE); BIALASINSKI, Leszek, D-40880 Ratingen (DE); SCHRADER, Karlheinz, D-37639 Bevern (DE)
(86) Internationale Anmeldenummer: EP9900085
(87) Internationale Veröffentlichungsnummer: WO9936051

(56) Entgegenhaltungen:
- EP-A- 0 532 465
- WO-A-90/05521
- WO-A-94/18944
- WO-A-94/21222
- WO-A-95/11663

## Beschreibung

Die Erfindung betrifft kosmetische und pharmazeutische Cremes in Form von Öl-in-Wasser-Emulsionen polarer Ölkomponenten, die als Emulgatoren pflanzliche Proteine oder proteinreiche Mehle von Pflanzensamenkernen enthalten. Solche Cremes lassen sich ohne die Verwendung üblicher ionischer oder hydrophiler nichtionischer Emulgatoren formulieren und weisen daher eine besonders hohe Pflegewirkung auf die Haut auf.

Der Einsatz von Sojaprotein in Kombination mit üblichen ethoxylierten Emulgatoren und Phospholipiden zur Herstellung von Lipoprotein-Emulsionen, z.B. für die Nahrungsmittelund Backwaren-Industrie ist aus US 4,360,537 bekannt. Auch in kosmetischen Emulsionen sind Proteine des öfteren als Emulgatoren beschrieben worden. So ist z.B. aus WO 94/21222 A1 bekannt, proteinhaltige Mehle von Pflanzensamen als Emulgatoren für Sonnenschutz-Lotionen zu verwenden. Allerdings weisen solche Emulsionen, wenn sie nicht zusätzlich hydrophile Emulgatoren enthalten, nur eine sehr begrenzte Stabilität auf. EP 0 532 465 A1 offenbart eine kosmetische Öl-in-Wasser-Emulsion, die Isopropylmyristat, ein Pflanzenprotein aus Sojabohnen, Cetylalkohol und einen hydrophilen Emulgator enthält.

Die vorliegende Erfindung beruht auf der Beobachtung, daß bestimmte pflanzliche Proteine in wäßriger Lösung nicht nur die Oberflächenspannung des Wassers, d.h. die Grenzflächenspannung Wasser/Luft, senken, sondern auch die Grenzflächenspannung von Wasser zu einer angrenzenden Ölphase. Wenn die Ölphase eine gewisse Polarität aufweist, d.h. wenn die Grenzflächenspannung Wasser/Öl unterhalb 30 mN/m (25° C) liegt, lassen sich mit solchen Proteinpräparaten sehr stabile Öl-in-Wasser-Emulsionen herstellen. Die Aufgabe der vorliegenden Erfindung bestand also darin, geeignete Proteinpräparate und darauf abgestimmte Ölkomponenten zu finden, die aufgrund ihrer Grenzflächeneigenschaften auch ohne Mitwirkung üblicher O/W-Emulgatoren die Herstellung stabiler Emulsionen möglich machen. Die dafür geeigneten pflanzlichen Proteine werden aufgrund ihrer lipophilen Eigenschaften im folgenden auch als Lipoproteine bezeichnet.

Gegenstand der Erfindung sind kosmetische und pharmazeutische Cremes in Form einer Öl-in-Wasser-Emulsion, die als Ölkomponenten wenigstens ein polares Öl, dessen Grenzflächenspannung gegen Wasser bei 25° C (γ¹) unterhalb von 30 mN/m (Millinewton pro Meter) liegt und die als Emulgator wenigstens ein Pflanzenprotein enthält, dessen 1 Gew.-%ige wäßrige Lösung (oder Dispersion) gegenüber der Ölkomponente eine Grenzflächenspannung bei 25° C (γ²) aufweist, die niedriger ist als die Grenzflächenspannung γ¹ zwischen Öl und Wasser.

Als kosmetische Cremes werden solche Zusammensetzungen verstanden, die aufgrund der enthaltenen Ölkomponente oder der in der Ölkomponente oder der wäßrigen Phase gelösten Wirkstoffe eine kosmetische Wirkung auf die Haut oder Haare entfalten. So weisen bestimmte Ölkomponenten eine hautweichmachende Wirkung auf. Geeignete öllösliche kosmetische Wirkstoffe sind z.B. Ceramide oder Ceramidanaloga, Vitamine, z.B. Tocopherole oder Tocopherolester, Retinolester, z.B. Retinolpalmitat, Sterine, Bisabolol, Duftstoffe, öllösliche Sonnenschutzmittel, Sebostatika und andere, die sensorischen Eigenschaften der Haut oder der Haare verbessernden oder die Haut oder Haare schützenden Stoffe. Geeignete wasserlösliche kosmetische Wirkstoffe sind z.B. Harnstoff, Allantoin, wasserlösliche Vitamine (Ascorbinsäure), Magnesiumsalze, Zucker und Polyole wie z.B. Glycerin, Sorbit oder Propylenglycol und wasserlösliche Pflanzenextrakte. Schließlich weisen auch die wasserlöslichen Proteine selbst eine kosmetisch günstige Hautwirkung auf, indem sie zur Bildung nichtfettender Lipoprotein-Filme auf der Haut mit gutem Wasserrückhaltevermögen führen.

Darüber hinaus können die erfindungsgemäßen Cremes auch öllösliche oder wasserlösliche pharmazeutische oder dermatologische Wirkstoffe zur Behandlung von Krankheiten der Haut oder Kopfhaut enthalten.

Besonders gut eignen sich als Ölkomponenten solche Öle oder Gemische von Ölen, die eine Grenzflächenspannung (γ¹) zu Wasser bei 25° C im Bereich von 5 bis 20 mN/m, bevorzugt von 6 bis 15 mN/m aufweisen, also Öle mit einer mittleren bis höheren Polarität.

Geeignete Ölkomponenten sind solche mit einer Ester-Gruppe im Molekül, z.B. die Fettsäureester einwertiger C₂-C₁₈-Alkohole wie z.B. Ethylstearat, Isopropylstearat, Isopropylmyristat, Butylstearat, Hexyllaurat oder Stearylisononanoat, auch Dicarbonsäureester wie z.B. Di-n-butyl-adipat oder Diisooctylsuccinat.

Bevorzugt sind als Ölkomponenten eine oder mehrere aus der Gruppe der C₈-C₂₂-Fettsäureester einwertiger oder mehrwertiger C₂-C₆-Alkohole oder der C₁₂-C₂₂-Alkoholester einwertiger oder mehrwertiger C₂-C₆-Hydroxycarbonsäuren oder Gemische davon enthalten.

Als C₈-C₂₂-Fettsäureester mehrwertiger C₂-C₆-Alkohole eignen sich z.B. die als pflanzliche und tierische Öle vorkommenden Fettsäuretriglycerid-Öle, z.B. Olivenöl, Sonnenblumenöl, Sojaöl, Sesamöl, Haselnußöl, Mandelöl, Distelöl. Weniger geeignet ist z.B. Nachtkerzenöl, dessen Polarität so hoch ist, daß es selbst eine beträchtliche Grenzflächenaktivität zeigt (siehe Beispiel 07 auf Seite 7). Weiter geeignete Ester dieses Typs sind die Fettsäureester des 1,2-Propylenglycols, des Neopentylglycols, des Trimethylolpropans und des Pentaerythrits.

Als C₈-C₂₂-Fettalkoholester einwertiger oder mehrwertiger Hydroxycarbonsäuren sind vor allem die Ester der Glycolsäure, Milchsäure, Äpfelsäure, Weinsäure und Citronensäure geeignet.

Solche Ester auf Basis linearen C_{14/15}-Alkanolen und von in 2-Position verzweigten C_{12/13}-Alkanolen sind unter dem Warenzeichen Cosmacol®-Ester (Hersteller: Eni Chem. Augusta Industriale) von der Fa. Nordmann, Rassmann GmbH & Co, Hamburg, zu beziehen. Diese Ester eignen sich nicht nur besonders gut zur Herstellung der erfindungsgemäßen Lipoprotein-Cremes, sondern weisen darüber hinaus auch weitere sehr erwünschte hautkosmetische Wirkungen auf, z.B. beschleunigen sie die Zellerneuerung und verlangsamen die Hautalterung.

Die erfindungsgemäßen Cremes enthalten als Emulgator ein grenzflächenaktives Pflanzenprotein (Lipoprotein), das in 1 Gew.-%iger Lösung in Wasser gegenüber der Ölkomponente eine Erniedrigung der Grenzflächenspannung Öl/Wasser bewirkt. Bevorzugt sind solche Pflanzenproteine enthalten, die in 1 Gew.-%iger Lösung bei 25° C die Grenzflächenspannung des Wassers zur Ölkomponente um einen Betrag (γ¹ - γ²) von wenigstens 2 mN/m senken.

Als geeignete Pflanzenproteine, die diese Anforderungen bei den erfindungsgemäß geeigneten Ölkomponenten erfüllen, haben sich vor allem Proteine aus Hafer, Erbsen, Soja und Weizen erwiesen. Es ist aber davon auszugehen, daß viele Proteine aus Pflanzensamen, z.B. Mandelprotein, Haselnußprotein und vor allem Getreideproteine in gleicher Weise geeignet sind.

Die erfindungsgemäß geeigneten Proteine können von unterschiedlicher Reinheit sein. Der Proteingehalt sollte aber bei wenigstens 10 Gew.-% oder darüber liegen. Reinigungs- und Isolationsverfahren für pflanzliche Proteine sind vielfach in der Patentliteratur beschrieben. So ist z.B. aus EP 620 979 A1 ein Verfahren zur Herstellung fettarmer Haferprotein-Lösungen bekannt. Aus EP 371 601 A2 ist ein anderes Verfahren zur Isolierung einer tensidartigen Proteinfraktion aus Hafer bekannt. Ein Verfahren zur Herstellung eines Proteinkonzentrats mit einem Gehalt von 10 - 20 Gew.-% Protein durch Extraktion lipidischer Begleitstoffe mit Hexan ist auch in WO 94/21222 A1 beschrieben.

Es ist nicht erforderlich, daß das erfindungsgemäß geeignete Protein klar wasserlöslich ist. Es reicht vielmehr völlig aus, wenn sich so viel des Proteins bei 25° C in Wasser löst, daß der Gehalt an gelöstem Protein die Grenzflächenspannung zu der enthaltenen Ölkomponente um wenigstens 2 mN/m gegenüber reinem Wasser löst. Üblicherweise beträgt der Gehalt an wasserlöslichem bzw. in Wasser gelöstem Protein wenigstens 0,1 Gew.-% der wäßrigen Phase.

Bevorzugt werden ca. 1 - 10 Gew.-% des Proteins oder proteinhaltigen Mehls, bezogen auf die erfndungsgemäße Creme, eingesetzt. Die Ölkomponenten sind bevorzugt in einer Menge von 1 - 20 Gew.-% in der erfindungsgemäßen Creme enthalten.

Als weitere Hilfsmittel können die erfindungsgemäßen Lipoprotein-Cremes übliche galenische Hilfsstoffe in den gebräuchlichen Mengen enthalten. Solche Hilfsstoffe sind z.B. Verdickungsmittel für die wäßrige Phase, z.B. vom Typ der Pflanzengumme, der wasserlöslichen Celluloseether, der wasserlöslichen Biopolymeren oder der wasserlöslichen synthetischen Polymeren, der Schichtsilikate oder der pyrogenen Kieselsäuren. Weitere Hilfsstoffe sind z.B. Polyole oder Glycole wie z.B. Glycerin, 1,2-Propylenglycol, 1,3-Butylenglycol oder Sorbit, Konservierungsstoffe wie z.B. Pentandiol-1,5, Phenoxyethanol, p-Hydroxybezoesäureester, Glycin oder Sorbinsäure, Komplexbildner (z.B. Trilon B), Antioxidantien sowie Farb- und Duftstoffe.

Obwohl in vielen Fällen nicht erforderlich, kann es vorteilhaft sein, zur Erhöhung der Emulsionsstabilität Coemulgatoren vom Typ der polaren Fettstoffe oder der Wasser-in-Öl-Emulgatoren zusätzlich einzusetzen. Bevorzugt sind als Coemulgatoren solche aus der Gruppe der C₁₂-C₂₂-Fettalkohole, z.B. Cetyl-/Stearylalkohol oder der C₁₂-C₂₂-Fettsäurepartialester von C₂-C₆-Polyolen in einer Menge von 0,1 - 1 Gewichtsteilen pro Gewichtsteil der Ölkomponente enthalten.

Wasserlösliche Tenside oder Emulgatoren vom O/W-Typ sind hingegen zur Stabilisierung der erfindungsgemäßen Cremes nicht erforderlich und bevorzugt sind diese im wesentlichen frei von ionischen und hydrophilen Emulgatoren mit einem HLB-Wert von 5 oder darüber.

Die folgenden Beispiele sollen den Gegenstand der Erfindung näher erläutern ohne ihn hierauf zu beschränken.

### Beispiele

### 1. Untersuchung der Grenzflächenspannung zwischen Ölkomponenten und Wasser bzw. 1 Gew.-%igen Lösungen von Proteinen in Wasser

Es wurden die folgenden Handelsprodukte verwendet:

| **Proteinpräparate:** | | |
|---|---|---|
| P1 | Tech-O-6-020 MC | hydrolysiertes Haferprotein |
| P2 | COS-152-11-A | Haferprotein |
| P3 | COS-152-15-A | Weizenprotein |
| P4 | Tech-O-6-40-L | Hafer-Proteinextrakt |
| P5 | COS-151-2-A | Erbsenprotein |
| P6 | Tech-O-11-070 | Hafermehl (ca. 13 Gew.-% Protein). |

| **Ölkomponenten:** | |
|---|---|
| Myritol®PC | Propylenglycol Dicaprylate/Dicaprate |
| Kessco®IPS | Isopropylstearat |
| IPIS | Isopropyl-Isostearat |
| Cetiol®MM | Myristylmyristat |

| **Gemischte Ölkomponente:** | |
|---|---|
| Cosmacol®PLG | Gemisch aus Di-C₁₂₋₁₃-Alkyl-Tartrat, Tri-C₁₂₋₁₃-Alkyl-Citrat und Kieselsäure |

| **Zusammensetzung weiterer Ölkomponenten (in Gewichtsteilen)** | | | | | | | | |
|---|---|---|---|---|---|---|---|---|
| | **01** | **02** | **03** | **04** | **05** | **06** | **07** | **08** |
| Myritol PC | 1 | 1 | 1 | 1 | 1 | 1 | 1 | 1 |
| Kessco IPS | 2 | 2 | - | 2 | 2 | 2 | 2 | 1 |
| IPIS | - | - | 2 | - | - | - | - | - |
| Cosmacol PLG | 1 | 1 | 1 | 1 | 1 | 1 | 1 | - |
| Distelöl | - | 1 | 1 | - | - | - | - | - |
| Mandelöl | - | - | - | 1 | - | - | - | - |
| Sesamöl | - | - | - | - | 1 | - | - | - |
| Rizinusöl | - | - | - | - | - | 1 | - | - |
| Nachtkerzenöl | - | - | - | - | - | - | 1 | - |

| **Messung der Grenzflächenspannung:** | | |
|---|---|---|
| **Ölkomponente** | **Grenzflächenspannung (mN/m) 25° C** | |
| | **Wasser (γ**^{**1**}**)** | **1 Gew.-% Protein in Wasser (γ**^{**2**}**)** |
| Kessco IPS | 27,8 | 17,7 (Protein P1) |
| | | 15,5 (Protein P2) |
| | | 15,8 (Protein P3) |
| | | 20,7 (Protein P4) |
| | | 11,4 (Protein P5) |
| | | 19,2 (Protein P6) |
| 01 | 7,1 | 3,8 (Protein P6) |
| 02 | 10,8 | 5,1 (Protein P6) |
| 03 | 10,9 | 4,4 (Protein P6) |
| 04 | 10,4 | 3,9 (Protein P6) |
| 05 | 10,8 | 4,1 (Protein P6) |
| 06 | 14,1 | 5,9 (Protein P6) |
| 07 | 4,5 | 4,7 (Protein P6) |
| 08 | 24,8 | Protein P1 : 14,0 |
| | | Protein P2 : 15,3 |
| | | Protein P3 : 13,4 |
| | | Protein P4 : 18,6 |
| | | Protein P5 : 8,9 |
| | | Protein P6 : 9,4 |

Die Messungen wurden mit einem Ringtensiometer durchgeführt.

### 2. Beispiele für erfindungsgemäße Cremes (1 - 8):

| | |
|---|---|
| Ölkomponente 01 bis 08 | 3,0 Gew.-% |
| Myritol PC | 3,5 Gew.-% |
| Kessco IPS | 6,0 Gew.-% |
| Cosmacol PLG | 3,0 Gew.-% |
| Cetiol MM | 2,5 Gew.-% |
| Cutina MD-A | 2,5 Gew.-% |
| Stenol 16/18 | 2,0 Gew.-% |
| Crodamol PMP | 1,0 Gew.-% |
| Protein P6 oder P5 | 3,0 Gew.-% |
| 1,3-Butylenglycol | 10,0 Gew.-% |
| Glycin | 1,0 Gew.-% |
| Trilon B (EDTA) | 0,2 Gew.-% |
| Wasser | Rest zu 100% |

Es wurden folgende Coemulgatoren verwendet:

| | | |
|---|---|---|
| (1) | Cutina®MD-A | Glycerin-Mono-/Di-palmitat/stearat |
| (2) | Stenol®1618 | Cetyl-/Stearylalkohol (1 : 2) |
| (3) | Crodamol®PMP | PPG-2-Myristylether-propionat |

### Herstellung:

Das Proteinpräparat wurde in 60 - 80° C warmem Wasser unter Verwendung eines Homogenisators (Ultraturax) gelöst (oder gequollen). Dann wurde die Proteinquellung mit der auf 60 - 80° C erwärmten Wasserphase (Wasser, 1,3-Butylenglycol, Glycin, Trilon B) unter Rühren vereinigt.

Die Ölkomponenten und Coemulgatoren wurden gemischt und auf ca. 80° C erwärmt. Schließlich wurde die Ölphase mit der heißen Wasserphase vereinigt und emulgiert.

Es bildete sich eine stabile Emulsion, nach dem Abkühlen auf 20° C eine glatte Creme von hoher Stabilität. Mit der Ölkomponente 07 bildete sich eine instabile Creme.

## Patentansprüche

1. Kosmetische und pharmazeutische Cremes in Form einer Öl-in-Wasser-Emulsion, die als Ölkomponente wenigstens ein polares Öl, dessen Grenzflächenspannung gegen Wasser bei 25° C (γ¹) unterhalb 30 mN/m liegt, und als Emulgator wenigstens ein Pflanzenprotein enthält, dessen 1 gew.%-ige wäßrige Lösung gegenüber der Ölkomponente eine Grenzflächenspannung bei 25° C (γ²) aufweist, die niedriger ist als die Grenzflächenspannung γ¹ zwischen Öl und Wasser, und die frei sind von ionischen und hydrophilen Emulgatoren mit HLB-Werten von 5 und darüber.

2. Kosmetische und pharmazeutische Cremes gemäß Anspruch 1, **dadurch gekennzeichnet, daß** als polare Ölkomponente ein Öl oder ein Gemisch von Ölen enthalten ist, das eine Grenzflächenspannung (γ¹) zu Wasser bei 25° C im Bereich von 5 bis 20 mN/m, bevorzugt von 6 bis 15 mN/m, aufweist

3. Kosmetische und pharmazeutische Cremes gemäß Anspruch 1 oder 2, **dadurch gekennzeichnet, daß** als Emulgator ein Pflanzenprotein enthalten ist, das in 1 Gew.-%iger Lösung bei 25° C die Grenzflächenspannung des Wassers zur Ölkomponente um einen Betrag (γ¹ - γ²) von wenigstens 2 mN/m senkt.

4. Kosmetische und pharmazeutische Cremes nach einem der Ansprüche 1 bis 3, **dadurch gekennzeichnet, daß** als Protein ein Protein aus Hafer, Weizen oder Erbsen oder ein proteinreiches Mehl dieser Pflanzensamen mit einem Gehalt von mehr als 10 Gew.-% Protein enthalten ist.

5. Kosmetische oder pharmazeutische Cremes nach einem der Ansprüche 1 bis 4, **dadurch gekennzeichnet, daß** als Ölkomponenten eine oder mehrere aus der Gruppe der C₈-C₂₂-Fettsäureester einwertiger oder mehrwertiger C₂-C₆-Alkohole oder der C₁₂-C₂₂-Alkanolester ein- oder mehrwertiger C₂-C₆-Hydroxycarbonsäuren oder deren Gemische enthalten sind.

6. Kosmetische oder pharmazeutische Cremes nach einem der Ansprüche 1 bis 5. **dadurch gekennzeichnet, daß** sie als Coemulgatoren solche aus der Gruppe der C₁₂-C₂₂-Fettalkohole oder der C₁₂-C₂₂-Fettsäurepartialester von C₂-C₆-Polyolen in einer Menge von 0,1 - 1 Gewichtsteilen pro Gewichtsteil der Ölkomponente enthalten.

## Claims

1. Cosmetic and pharmaceutical creams in the form of an oil-in-water emulsion which contain at least one polar oil, of which the interfacial tension with water at 25°C (γ¹) is below 30 mN/m, as oil component and at least one vegetable protein, of which a 1% by weight aqueous solution has an interfacial tension with the oil component at 25°C (γ²) that is lower than the interfacial tension γ¹ between oil and water, as emulsifier and which are free from ionic and hydrophilic emulsifiers with HLB values of 5 or more.

2. Cosmetic and pharmaceutical creams as claimed in claim 1, **characterized in that** the polar oil component is an oil or a mixture of oils which has an interfacial tension (γ¹) with water at 25°C in the range from 5 to 20 mN/m and preferably in the range from 6 to 15 mN/m.

3. Cosmetic and pharmaceutical creams as claimed in claim 1 or 2, **characterized in that** the emulsifier is a vegetable protein of which a 1% by weight solution reduces the interfacial tension between water and the oil component at 25°C by an amount (γ¹ - γ²) of at least 2 mN/m.

4. Cosmetic and pharmaceutical creams as claimed in any of claims 1 to 3, **characterized in that** a protein of oats, wheat or peas or a protein-rich flour of these plant seeds containing more than 10% by weight protein is present as the protein.

5. Cosmetic or pharmaceutical creams as claimed in any of claims 1 to 4, **characterized in that** the oil components present are one or more oil components from the group of C₈₋₂₂ fatty acid esters of monohydric or polyhydric C₂₋₆ alcohols or C₁₂₋₂₂ alkanol esters of monobasic or polybasic C₂₋₆ hydroxycarboxylic acids or mixtures thereof.

6. Cosmetic or pharmaceutical creams as claimed in any of claims 1 to 5, **characterized in that** they contain co-emulsifiers from the group of C₁₂₋₂₂ fatty alcohols or C₁₂₋₂₂ fatty acid partial esters of C₂₋₆ polyols in a quantity of 0.1 to 1 part by weight per part by weight of the oil component as co-emulsifiers.

## Revendications

1. Crèmes cosmétiques et pharmaceutiques sous forme d'une émulsion huile dans l'eau qui contiennent comme composant huileux au moins une huile polaire dont la tension interfaciale par rapport à l'eau, à 25°C (γ¹) se situe en dessous de 30 mN/m et qui contiennent comme agent émulsionnant au moins une protéine végétale dont la solution aqueuse à 1 % en poids possède par rapport aux composants huileux, une tension interfaciale à 25°C (γ²) qui est plus basse que la tension interfaciale (γ¹) entre l'huile et l'eau, et qui sont dépourvues d'agents émulsionnants ioniques et hydrophiles ayant des valeurs de HLB de 5 et au-dessus.

2. Crèmes cosmétiques et pharmaceutiques conformément à la revendication 1,
**caractérisées en ce que**
comme composant huileux polaire une huile ou un mélange d'huile est contenu qui possède une tension interfaciale (γ¹) pour l'eau à 25°C dans la zone de 5 à 20 mN/m, de préférence de 6 à 15 mN/m.

3. Crèmes cosmétiques et pharmaceutiques conformément à la revendication 1 ou à la revendication 2,
**caractérisées en ce que**
comme agent émulsionnant une protéine végétale est contenue qui, en solution à 1 % en poids à 25°C abaisse la tension interfaciale de l'eau par rapport au composant huileux, d'un montant (γ¹-γ²) d'au moins 2 mN/m.

4. Crèmes cosmétiques et pharmaceutiques selon l'une quelconque des revendications 1 à 3.
**caractérisées en ce que**
comme protéine, une protéine provenant de l'avoine, du blé ou des pois ou une farine riche en protéines de ces graines de plantes ayant une teneur de plus de 10 % en poids de protéines, est contenue.

5. Crêmes cosmétiques et pharmaceutiques selon l'une quelconque des revendications 1 à 4,
**caractérisées en ce que**
comme composants huileux un ou plusieurs choisis dans le groupe des esters d'acide gras en C₈-C₂₂ d'alcools en C₂-C₆ monofonctionnels ou plurifonctionnels ou des esters d'alkanol en C₁₂-C₂₂ d'acides hydroxy-carboxyliques en C₂-C₆ mono- ou plurifonctionnels ou leurs mélanges sont contenus.

6. Crèmes cosmétiques et pharmaceutiques selon l'une quelconque des revendications 1 à 5,
**caractérisées en ce qu'**
elles contiennent comme agents co-émulsionnants ceux choisis dans le groupe des alcools gras en C₁₂-C₂₂ ou des esters partiels d'acides gras en C₁₂-C₂₂ de polyols C₂-C₆ en une quantité de 0,1-1 partie en poids par partie en poids des composants huileux.
